(19) Europäisches Patentamt
European Patent Office
Office européen des brevets

(11) Publication number: 0 096 356
B1

(12) **EUROPEAN PATENT SPECIFICATION**

(45) Date of publication of patent specification: **21.09.88**

(51) Int. Cl.⁴: **C 12 P 13/00**, C 07 C 129/12, C 12 N 1/20, A 61 K 31/195 // C12R1/01

(21) Application number: **83105416.8**

(22) Date of filing: **01.06.83**

# (54) The novel microorganism strain Chromobacterium violaceum, pharmaceutically active compounds being produced by the strain, a process for preparing the compounds and the use of these compounds as medicaments.

(30) Priority: **07.06.82 JP 96276/82**

(43) Date of publication of application:
**21.12.83 Bulletin 83/51**

(45) Publication of the grant of the patent:
**21.09.88 Bulletin 88/38**

(84) Designated Contracting States:
**AT BE CH DE FR GB IT LI NL SE**

(56) References cited:
**GB-A-2 005 687**
**JP-A-55 102 597**

**PATENTS ABSTRACTS OF JAPAN, Vol. 4, Nr. 155, (C-29) (637), October 29, 1980.**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao**
**4 chome-23, Toyotama-kita**
**Nerima-ku Tokyo (JP)**
Inventor: **Aoyagi, Takaaki**
**3-3-6, Honkugenuma**
**Fujisawa-city Kanagawa Prefecture (JP)**
Inventor: **Takeuchi, Tomio**
**5-1-11, Higashi-gotanda**
**Shinagawa-ku Tokyo (JP)**
Inventor: **Hamada, Masa**
**1-26, Naito-cho**
**Shinjuku-ku Tokyo (JP)**
Inventor: **Ishizuka, Masaaki**
**3-17, Denenchofu-honcho**
**Ohta-ku Tokyo (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 096 356 B1

**Description**

The present invention relates to the novel microorganism strain Chromobacterium violaceum FERM—P—6521 the novel pharmaceutically active substance Arphamenine a process for producing the same by cultivating the aforementioned strain and further to its use as a medicament, particularly as immunopotentiator and anti-cancer drug. The substance Arphamenine which refers to Arphamenine A or Arphamenine B (as explained below) or both of these Arphamenines in general or a mixture comprising both Arphamenines has been found to exhibit activity of augmenting cell-mediated immunity and anti-cancer activity. Particularly, it has been found that the substance according to the present invention is effective in inhibiting the decomposition of arginine-β-naphthylamide by amino-peptidase B.

The novel substance Arphamenine has the following formula

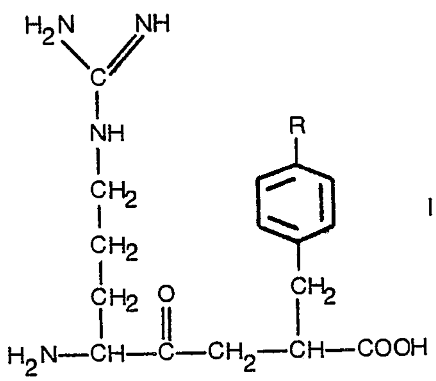

I

where R represents a hydrogen atom in the case of Arphamenine A or a hydroxyl group in the case of Arphamenine B,
or their salts.

As the later-given Examples will reveal, Arphamenines A and B can be each obtained as colorless powder by fractionating the filtrate of a culture medium after cultivation of an Arphamenine-producing bacterium. The fractionation may be carried out by chromatography on CM-Sephadex, etc. The properties of the resulting fractions will be mentioned below.

Arphamenine A has a melting point of 117° to 119°C and a molecular weight, determined by mass spectrometry, of 320. The elemental analysis values are C: 53.45%, H: 7.11%, N: 14.91%, O: 14.20%, Cl: 10.49%, indicating the molecular formula $C_{16}H_{24}N_4O_3 \cdot HCl$. The ultraviolet absorption spectrum for an aqueous solution containing 100 γ/ml, of Arphamenine A is illustrated in Fig. 1, which shows an absorption with λ max of 257 nm (ε 180). The infrared absorption spectrum of Arphamenine A by the KBr method is shown in Fig. 2, which reveals characteristic absorption bands at 3370, 3170, 2950, 1730, 1670, 1560, 1460, 1410, 1320, 1190, 1110, 760 and 710 $cm^{-1}$. The nuclear magnetic resonance absorption spectrum ($^1$H-NMR) (solution in heavy water, δ, 100 MHz) is given in Fig. 3 showing absorptions at 2.04—2.33 ($CH_2$), 2.35—2.72 ($CH_2$), 3.34—3.69 ($CH_2 \times 2$), 3.69—3.84 (CH, $CH_2$), 4.83 (CH), and 7.82—8.00 ($C_6H_5$) δ.

Arphamenine B has a melting point of 118—120°C and a molecular weight, determined by mass spectrometry, of 336. The elemental analysis values are C: 49.30%, H: 6.88%, N: 13.77%, O: 20.82% and Cl: 8.73%, indicating the molecular formula $C_{16}H_{24}N_4O_4 \cdot HCl \cdot H_2O$. The ultraviolet absorption spectrum for an aqueous solution containing 100 γ/ml of Arphamenine B is given in Fig. 4, which shows absorptions with λ max of 275 nm (ε 1040) and λ max of 222 nm (ε 5500). The infrared absorption spectrum of Arphamenine B by the KBr method is indicated in Fig. 5 which shows characteristic absorption bands at 3370, 3180, 2960, 1730, 1670, 1560, 1520, 1460, 1420, 1330, 1250, 1180, 1110 and 840 $cm^{-1}$. The nuclear magnetic resonance absorption spectrum (solution in heavy water, δ, 100 MHz) is revealed in Fig. 6 showing absorptions at 1.87—2.30 ($CH_2$), 2.30—2.62 ($CH_2$), 3.14—3.54 ($CH_2 \times 2$), 3.54—3.78 (CH, $CH_2$), 4.75 (CH), 7.30—7.67 ($C_6H_5$) δ.

The present invention relates further to a process for producing the novel physiologically active substance Arphamenine A and/or the novel physiologically active substance Arphamenine B, which comprises cultivating an Arphamenine-producing microorganism belonging to the genus *Chromobacterium* to cause said substance(s) to be produced and accumulated in the culture medium, and then recovering said substance(s) from the culture medium after cultivation.

In the process of this invention, the Arphamenine-producing microorganism means a microorganism capable of producing Arphamenine A or Arphamenine B or both of these Arphamenines. An example of the microorganism is *Chromobacterium violaceum* BMG361—CF4, a strain that was isolated from soil collected at Poropinai on the shore of Lake Shikotsu, Hokkaido, Japan. This strain was deposited on May 4, 1982 at the Fermentation Research Institute, Agency of Industrial Science and Technology, Ministry of International Trade and Industry of Japan under Deposit No. 6521 (FERM P—6521).

The microbiological characteristics of the above-mentioned strain are as follows:

(a) Morphological characteristics
(1) Shape and size of the cell: Rod; about 0.8 to 1.0 microns × 2.0 to 4.0 microns
(2) Pleomorphism: Non-pleomorphic
(3) Motility/flagellation: Motile/polar and lateral flagella
(4) Sporogenicity: Non-sporulating
(5) Gram's stain: Negative
(6) Acid-fastness: Negative

(b) Culture characteristics on various media (cultivation at 27°C, 20°C and 30°C for bouillon gelation stab culture only)
(1) Bouillon agar plate culture:
Colonies grown were somewhat elevated semispherically, and their margins were smooth and circular. The surfaces were smooth and glossy. After about 17 hours of cultivation, the colonies were translucent, but gradually became opaque and presented a rubbery appearance. Around the second day of cultivation, the colonies turned purple, but produced no diffusible pigments.
(2) Bouillon agar slant culture:
Colonies grew uniformly along the lines of inoculation. The surfaces were smooth and lustrous, and the margins were smooth. Around the second day of cultivation, colonies at the bottom of the slanting agar surface developed a purple color, not so soluble pigments were observed.
(3) Bouillon liquid culture:
Around the second day of cultivation, purple annular colonies were formed on the surface of the medium. After about 40 hours of cultivation, the number of strain cells at the bottom of the test tube increased. The cells turned purple around the third day of cultivation.
(4) Bouillon gelatin stab culture:
When the cultivation temperature was 30°C, cells grew along the line of stab. Around the third day of cultivation, the medium liquefied, and the area of liquefaction had a tubular form. In the case of cultivation at 20°C, liquefaction was seen on the sixth day of cultivation.
(5) BCP milk culture:
After 3 days of cultivation, BCP turned blue, and the medium coagulated. On the 5th day of cultivation, coagulation was completed, and immediately, peptonization began. Peptonization was completed in about 2 weeks.

(c) Physiological characteristics (the cultivation temperatures were all 27°C, unless otherwise specified)
(1) Reduction of nitrates: Formation of nitrites from nitrates
(2) Denitrification (method of Komagata et al.: Edited by Takeharu Hasegawa: Classification and Identification of Microorganisms, page 223, Tokyo University Publisher, 1975): Positive, no generation of gases
(3) MR test: Positive
(4) VP test: Negative
(5) Production of indole: Negative
(6) Production of hydrogen sulfide (TSI Agar Midium, a product of Eiken, Japan): Negative
(7) Hydrolysis of starch: Negative
(8) Utilization of citric acid: Positive on a Koser medium and a Christensen medium
(9) Utilization of inorganic nitrogen source (sodium sulfate, ammonium sulfate, and sodium glutamate): Any inorganic nitrogen sources were utilized for the growth.
(10) Production of pigment (King A and B Medium, products of Eiken): Tiny amounts of a yellow soluble pigment formed on each medium
(11) Urease (Uria Medium, a product of Eiken): Negative
(12) Oxidase: Positive
(13) Catalase: Positive
(14) Growth temperature and pH ranges: Grew at 12 to 37°C, most preferably about 27 to 30°C. Grew at a pH of 5.0 to 8.6, most preferably 6.0 to 7.8
(15) Oxgen demand: Aerobic (facultative anaerobic)
(16) O—F test (according to the Hugh & Leifson method): Fermentative
(17) Formation of acids and gases from saccharides (a Hugh & Leifson culture): Acids were formed from D-glucose, D-fructose and trehalose. No acids were formed from the following 19 saccharides: L-arabinose, D-xylose, D-mannose, D-galactose, maltose, sucrose, lactose, D-sorbitol, D-mannitol, inositol, glycerin, starch, adonitol, cellobiose, dulcitol, inulin, melibiose, melezitose, and raffinose.
None of the saccharides formed gases.
(18) Hydrolysis of casein: The microorganism was streak-cultured in a casein agar plate (pH 7.4) prepared by adding sterilized skim milk to a bouillon agar to a concentration of 5%, and solidifying the mixture. After 24 hours of cultivation, casein was digested, and its hydrolysis was completed on the 8th day of cultivation.

(19) Visible spectrum measurement on purple pigment: The purple cells grown in the bouillon agar slant were extracted with ethanol, and the extract was measured for its visible spectrum. Maximum absorption was seen at 573 nm, and minimum absorption at 430 nm.

When the pigment was dissolved in 10% ethanol sulfate, it formed a green solution with maximum absorption at 693 nm. This fact showed the purple pigment to be a violacein pigment (see Bergey's Manual of Determinative Bacteriology, 8th edition, p. 354).

A summary of the above-mentioned characteristics showed FERM—P—6521 to be a gram-negative facultative anaerobic bacillus having polar and lateral flagella. Its colonies contained a purple pigment, which was demonstrated to be violacein on measurement of its visible spectrum. Research on these properties was conducted by reference to Bergey's Manual of Determinative Bacteriology 8th edition, and the results presumed BMG361—CF4 Strain to belong to the genus *Chromobacterium. Chromobacterium* includes the two species, i.e., *C. violaceum* and *C. lividum.* FERM—P—6521 strains possessed properties very similar of those of the former species. In detail, FERM—P—6521 Strain was clearly distinguished from *C. lividum* in terms of growth at 37°C, patterns of acid formation from saccharides (acid production from trehalose; no acid production from arabinose or xylose), hydrolysis of casein, etc. Accordingly, FERM—P—6521 Strain was identified as *Chromobacterium violaceum* FERM—P—6521.

The process of this invention will be described in detail below. Known nutrients for bacteria can be used, if desired, to cultivate the Arphamenine-producing microorganism in carrying out the process of the present invention. Examples of carbon sources are commercially available fats and carbohydrates, such as glycerin, glucose, lactose, sucrose, starch, maltose and molasses. Examples of nitrogen sources include marketed goods, such as peptone, meat extract, corn steep liquor, cottonseed meal, peanut meal, soybean meal, corn gluten meal, fish meal, yeast extract, N—Z amine, casein, sodium nitrate, ammonium nitrate and ammonium sulfate. Examples of inorganic nutrients are sodium chloride phosphates, calcium carbonate and magnesium sulfate. Trace amounts of other metal salts may be added if desired.

Any of these cited materials may be used so long as they can be utilized by the Arphamenine-producing microorganism and they will not impede the production of the present substance.

Any known materials for bacterial cultivation can be used. Particularly preferred components of culture media are carbon sources including glycerin and soluble starch, and nitrogen sources including soybean meal, fish meal and corn gluten meal. Examples of preferred culture media are a medium containing 1.5% glycerin, 1.5% soluble starch, 0.5% Prorich, 1.5% fish meal and 0.2% calcium carbonate, and a medium containing 3% soluble starch, 0.5% Prorich, 1.2% corn gluten meal and 0.2% calcium carbonate.

Liquid culture is preferred for the mass-production of Arphamenine. The cultivation temperature can be selected within a range which permits the Arphamenine-producing microorganism to grow and produce Arphamenine. The particularly preferred temperature is 25 to 35°C. The cultivation is usually performed until a sufficient amount of the present substance is accumulated in the culture medium.

For example, a culture medium containing 3% soluble starch, 0.5% Prorich, 1.2% corn gluten meal and 0.2% calcium carbonate was sterilized, and then inoculated with a loopful of a slant culture of the Arphamenin-producing microorganism. The inoculum was aerobically shake-cultured at 27°C. After 8 to 52 hours of cultivation, Arphamenine was seen to be accumulated. Arphamenine was produced satisfactorily by cultivation in a tank as well as shake culture. For instance, 300 liters of the medium placed in a 570-liter fermentation vessel was sterilized, and the cultivation was conducted with stirring at 190 rpm under aeration with 300 liters/minute of sterilized air. Under these conditions, the production of the present substance reached its maximum level in 23 hours.

Follow-up of Arphamenine during its cultivation and purification was carried out by measuring the inhibitory activity against aminopeptidase B in the following way:

The aminopeptidase B inhibitory activity of Arphamenine was measured by a modification of the method described in V. K. Hoppusu, K. K. Makinen & G. G. Glenner, Archives of Biochemistry and Biophysics *114*, 557, 1966. In detail, 0.5 ml of 0.1 M tris-hydrochloride buffer solution (pH 7.0) and 0.25 ml of a solution containing the specimen were added to 0.25 ml of 0.002 M arginine-β-naphthylamide. The mixed solution was heated at 37°C for 3 minutes. To the heated solution was added 5 μl of a solution of aminopeptidase B that had been purified with DEAE-Cellulose according to the enzyme-purification technique of Hoppusu et al. The mixture was reacted for 30 minutes at 37°C, followed by adding to the reaction mixture 1 ml of 1.0 M acetic acid buffer solution (pH 4.2) containing Fast Garnet GBC (o-aminoazotoluene, diazonium salt) in a concentration of 1 mg/ml and Surfactant Tween 20 in a concentration of 10%. The resulting mixture was allowed to stand for 15 minutes at room temperature, and measured for absorbance (a) at 525 nm. Simultaneously, a blank absorbance (b) was obtained using an Arphamenine-free buffer solution only. Percent inhibition of aminopeptidase B was calculated from the equation $[(b - a)/b] \times 100$. According to this technique, 0.005 μg/ml of Arphamenine A and 0.002 μg/ml of Arphamenine B each inhibited 50% of aminopeptidase B activity ($IC_{50}$).

Arphamenine exists in culture media and cells of the Arphamenine-producing microorganism after cultivation. Arphamenine can be recovered in good yields by adsorbing a filtrate of the culture medium to an adsorbent and isolating the substance from the adsorbate. Examples of the adsorbent include organic adsorbents such as activated carbon, Amberlite XAD—4 and Diaion HP—20, ion exchange resins, alumina and silica gel. For example, Arphamenine is adsorbed to Amberlite XAD—4 and eluted with an aqueous solution of acetone. More specifically, a suitable column is packed with Amberlite XAD—4 in an amount of

1/10 the amount of a filtrate of the culture medium after cultivation. The filtrate containing Arphamenine is passed through the column and adsorbed thereto. The Amberlite XAD—4 column is washed with water, and eluted with a 50% aqueous solution of acetone whose amount is 2 to 4 times that of Amberlite XAD—4. The eluate contains more than 70% of Arphamenine contained in the culture filtrate. The eluate so obtained is concentrated to dryness at reduced pressure to obtain a crude powder of Arphamenine.

Chromatography on an ion-exchange resin can also be used in the purification. Particularly, chromatography on CM-Sephadex is efficient, making it possible to separate Arphamenines A and B from each other by a gradient elution using aqueous solution of sodium chloride. For instance, the crude powder that has been obtained by adsorption to Amberlite XAD—4 and elution with an aqueous solution of acetone is subjected to chromatography on CM-Sephadex. The column is eluted with an aqueous solution of salt such as sodium chloride to collect a fraction containing Arphamenine A and a fraction containing Arphamenine B separately. Then, Arphamenine A and B are isolated from each of them.

The final purification of the present substance can be performed by desalting with Sephadex LH—20.

Arphamenine of the present invention was examined for pharmacological effects. The present substance was found to potentiate cell-mediated immunity and possess an action of anti-cancer.

The biological activities of Arphamenine A and B of this invention are described by reference to the following experimental examples.

1. Effect on cell-mediated immunity in normal mice

The effects of Arphamenine A and B on cell-mediated immunity were investigated using as an index delayed-type hypersensitivity (D.T.H., for short) produced by inoculating sheep red blood cells (SRBC, for short) on the foot pads of mice as an antigen (see J. Exp. Med. 139, 1529—1539, 1974).

A suspension of $10^8$ SRBCs in 0.05 ml of physiological saline solution was inoculated subcutaneously into one of the foot pads of $CDF_1$ mice (female, 8 weeks old). Simultaneously, an aqueous solution containing 5, 0.5, 0.05 or 0.005 mg/kg of Arphamenine A or B was administered orally at a single dose. Four days after administration, a suspension of $10^8$ SRBCs in 0.05 ml of physiological saline solution was administered subcutaneously to the other foot pad to cause a secondary sensitization. Twenty-four hours later, swelling on the foot pad (increased thickness of the foot pad) was measured with a calipers. In control animals subcutaneously injected with SRBCs in physiological saline solution without administration of the test compound, the increased thickness of the foot pad was evaluated to be 100%. The increased thickness of the foot pad in the treated animals was compared with that in the untreated animals to calculate the ratio of the former value to the latter value (100%), thereby determining the cell-mediated immunity potentiating effect of the test compound. The results are revealed in the following tables:

Table 1

| Test compounds | Dose (mg/kg) | Increased thickness of foot pad (x 0.1 mm) | Ratio to the control value (%) |
|---|---|---|---|
| Arphamenine A | 5 | 10.4 ± 0.77 | 125 |
| Dito | 0.5 | 11.5 ± 1.29 | 139 |
| Dito | 0.05 | 13.0 ± 0.97 | 157 |
| Dito | 0.005 | 11.9 ± 1.00 | 143 |
| Bestatin* | 0.5 | 12.5 ± 1.24 | 151 |
| Control | | 8.3 ± 0.97 | 100 |

*Reference compound

Table 2

| Test compounds | Dose (mg/kg) | Increased thickness of foot pad (x 0.1 mm) | Ratio to the control value (%) |
|---|---|---|---|
| Arphamenine B | 5 | 13.7 ± 1.77 | 157 |
| Dito | 0.5 | 14.7 ± 1.74 | 169 |
| Dito | 0.05 | 14.0 ± 1.33 | 161 |
| Dito | 0.005 | 12.4 ± 1.40 | 143 |
| Bestatin* | 0.5 | 13.0 ± 1.13 | 149 |
| Control | | 8.7 ± 1.48 | 100 |

*Reference compound

2. Effect on cell-mediated immunity in mice with carcinoma

(1) Methods using SRBCs as antigen

Swelling on the foot pad in mice was investigated in the same way as in Experimental Example 1, except that $CDF_1$ mice were intraperitoneally inoculated with $10^6$ cells of Ascites Sarcoma 180 and that Arphamenine A was administered intraperitoneally once daily for 4 consecutive days, beginning on the day of sensitization SRBCs, and two days layer, SRBCs were given to cause a secondary sensitization. The results are shown in Table 3.

Table 3

| Test compounds | Dose (mg/kg) | Increased thickness of foot pad (x 0.1 mm) | Ratio to the control value (%) |
|---|---|---|---|
| Arphamenine A | 5 | 5.8 ± 1.19 | 129 |
| Dito | 0.5 | 6.7 ± 1.03 | 149 |
| Dito | 0.05 | 6.5 ± 1.29 | 144 |
| Dito | 0.005 | 5.9 ± 0.79 | 131 |
| Bestatin* | 5 | 6.0 ± 0.96 | 133 |
| Control | | 4.5 ± 0.74 | 100 |

* Reference compound

(2) Method using picryl chloride as antigen

In mice with Ascites Sarcoma 180, the effect of Arphamenine A on cell-mediated immunity was investigated in the following manner observing D.T.H. reaction produced with the use of picryl chloride as antigen. $10^6$ cells of Ascites Sarcoma 180 were transplanted intraperitoneally into 12-week-old female $CDF_1$ mice (6 mice/group). The day of transplantation was designated as Day 0. On Day 1, a shaved area (25 mm × 15 mm) of the abdomen was sensitized with 0.6 ml of a 6% ethanol solution of picryl chloride absorbed to a cut absorbent cotton (20 mm × 20 mm × 2 mm). On Day 8, the thickness of the auricles of both ears was measured with a dial gauge to obtain a baseline value (a). Then, both auricles were sensitized with a 1% olive oil solution of picryl chloride absorbed to a cut absorbent cotton of the size 10 mm × 4 mm × 1 mm. On Day 9, swelling on the sensitized auricles was measured with a dial gauge to obtain a value (b). The baseline value (a) was subtracted from the value (b) to determine an increase in the thickness of the auricles (b − a) in the control group.

Separately, 0.5, 0.05 or 0.005 mg/kg of the test compound dissolved in physiological saline solution was orally administered 6 times/day for consecutive days from Day 1 to Day 8 inclusive, and picryl chloride sensitization was carried out in the same way as in the control group. The increase in the thickness of both auricles (b′ − a′) in this treated group was determined in the same way as in the control group.

The ratio of such increase for the treated group to that for the control group was calculated from the following equation

$$\frac{(b' - a')}{(b - a)} \times 100$$

with the value for the control group set at 100%, thereby determining the activity of the test compound to potentiate cell-mediated immunity. The results are shown in Table 4.

Table 4

| Test compounds | Dose (mg/kg) | Increased thickness of auricles (x 0.1 mm) | Ratio to the control value (%) |
|---|---|---|---|
| Arphamenine A | 0.5 | 6.05 ± 0.97 | 136.0 |
| Dito | 0.05 | 7.40 ± 0.72* | 166.3 |
| Dito | 0.005 | 6.40 ± 0.63 | 143.8 |
| Controll** | | 4.45 ± 2.36 | 100.0 |

* $P < 0.05$

** Physiological saline solution

The above results demonstrate that a significant activity to potentiate cell-mediated immunity was obtained in the group receiving 0.05 mg/kg of Arphamenine A.

Experimental Example 3

Antitumor activity of Arphamenine against Ehrlich Solid Tumor

$3 \times 10^6$ cells of Ehrlich ascites carcinoma were transplanted subcutaneously into the flank of 8-week old female ddY mice (5 mice/group). The day of transplantation was designated as Day 0. Thereafter, 0.5, 0.05 or 0.005 mg/kg of the test compound dissolved in physiological saline solution was orally administered every other day over a total of 7 times until Day 15, beginning on Day 1. On Day 30, the size (shorter diameter$^2$ × longer diameter/2) and weight of tumor were measured. The results in this treated group were compared with those in the control group to calculate tumor inhibition rate (TIR, %) from the following equation:

$$\frac{C - T}{C} \times 100$$

in which C is the size or weight of tumor in the control group, and T is the size or weight of tumor in the treated group.

Results on TIR (%), an index to the antitumor effect of the test compound, are shown in the following table:

Table 5

| Test compounds | Dose (mg/kg) | Average size of tumor ($mm^3$) | TIR (%) | Average weight of tumor (g) | TIR (%) |
|---|---|---|---|---|---|
| Arphamenine A | 0.5 | 4467 ± 4311 | 15.8 | 2.62 ± 1.90 | 1.9 |
| Dito | 0.05 | 3120 ± 3584 | 41.2 | 1.66 ± 1.50 | 37.8 |
| Dito | 0.005 | 2378 ± 3138 | 55.2 | 1.62 ± 1.44 | 39.3 |
| Bestatin (reference compound) | 0.05 | 2089 ± 1946* | 60.6 | 1.47 ± 0.92 ** | 44.9 |
| Control | - | 5305 ± 2027 | 0 | 2.67 ± 0.84 | 0 |

* P < 0.1 (T-test)
**P < 0.05 (T-test)

The above results demonstrate that the administration in the amount of each 0.05 and 0.005 mg/kg of Arphamenine A produced a host-mediated antitumor effect in terms of tumor size and tumor weight.

Thus, Arphamenines A and B prove to potentiate cell-mediated immunity in normal animals, modulate cell-mediated immunity depressed by carcinoma, and exhibit a host-mediated antitumor effect.

Acute toxicity tests in mice have shown that no deaths are caused by Arphamenine A at an iv dose of 250 mg/kg or Arphamenine B at an iv dose of 150 mg/kg. Arphamenine is thus considered to be a safe substance.

As described above, Arphamenines A and B of the present invention each augment immunity and exhibit a host-mediated carcinostatic effect when administered singly. These compounds are therefore useful as immunopotentiators and antitumor immunomodulators or as adjuvants to various chemotherapeutic agents for use in the treatment of carcinomas.

Said drugs containing Arphamenine as active ingredients can be prepared by blending Arphamenine A or B or both Arphamenines A and B or their pharmaceutically acceptable salts, with the carriers in customary use, and if desired, further with various chemotherapeutic agents. Examples of the Arphamenine salts include salts formed through the reaction between the carboxyl group of Arphamenine and pharmaceutically acceptable cations, such as ammonium ions, and cations of alkali metals such as sodium and potassium, and cations of alkaline earth metals such as calcium and magnesium (i.e., carboxylates of Arphamenine). Additional examples include acid-addition salts formed through the reaction between the guanidyl or amino group of Arphamenine and pharmaceutically acceptable inorganic acids such as hydrochloric acid, or organic acids such as acetic acid. The compounds or drugs of this invention may be administered as oral preparations, injections or rectal suppositories. Lyophilized injections can be prepared by adding pH adjustors, buffers, stabilizers and excipients to said compounds comprising the active ingredients, and then freeze-drying the mixtures in customary manners. Injections for subcutaneous, intramuscular or intravenous administration can be prepared by adding pH adjustors, buffers, stabilizers, isotonizers and local anesthetics to the active ingredient-containing compounds, and then by applying customary procedures.

For the preparation of oral solids, excipients, and if desired, binders, disintegrators, lubricants, colorants, taste correctives and odor correctives may be added to the active ingredient-containing compounds, whereafter the mixture are formed into tablets, coated tablets, granules, powders and capsules by customary methods.

For the preparation of oral liquids, taste correctives, buffers, stabilizers and odor correctives may be added to the active ingredient-containing compounds, and then the mixtures are made into syrups and dry syrups by customary methods.

To prepare rectal suppositories, excipients, and if desired, surfactants may be added to the active ingredient-containing compounds, whereafter the mixtures are prepared into suppositories by customary technique.

The dose of Arphamenine may be varied depending on symptoms, but the usual adult dosage is 0.02 to 200 mg of Arphamenine once daily. When concomitant therapy with other chemotherapeutic agents for cancer or other immunopotentiators is to be instituted, Arphamenine in said dose range may be combined with these other drugs in their usual doses.

The production of Arphamenine of the present invention will be described in more detail with reference to the following working examples. However, this invention is in no way limited to these examples, since the physico-chemical properties of Arphamenine, and techniques for its production and purification clearly described by the present inventors would make it easy for anyone to modify the methods according to those disclosed throughout the present specification.

Example 1

A loopful of cells were taken from a slant culture of the Arphamenine-producing microorganism, *Chromobacterium violaceum* BMG361—CF4 (Deposit No. 6521 at the Fermentation Research Institute, Agency of Industrial Science and Technology, Ministry of International Trade and Industry of Japan).

The cells were inoculated into a culture medium containing 3% soluble starch, 0.5% Prorich, 1.2% corn gluten meal and 0.2% calcium carbonate. Said culture medium had been sterilized at 120°C for 20 minutes and dispensed in amounts of 110 ml in 500 ml rotary flasks. The inoculum was cultivated at 27°C at 180 rpm, and the output of Arphamenine was examined with the lapse of time. The Arphamenine output was maximal after 32 hours of cultivation. When 36 hours passed after initiating cultivation, the Arphamenine concentration evaluated by the anti-aminopeptidase B activity gradually decreased.

The pH of the culture medium during cultivation was 7.4 at the start of cultivation, 7.8 at 8 hours, 7.6 at 16 hours, 7.85 at 24 hours, 8.1 at 32 hours, 8.1 at 36 hours, and 8.45 at 52 hours.

Example 2

The strain, *Chromobacterium violacium* FERM—P—6521 was cultivated under the same conditions using the same culture medium as in Example 1. 9.5 liters of the culture medium obtained after cultivation was adjusted to a pH of 2 with hydrochloric acid, and suction-filtered with a filter aid (Hyflo Super Cell) to obtain 9 liters of a filtrate. The filtrate had an aminopeptidase B inhibitory activity ($IC_{50}$) of 0.05 μl/ml.

Example 3

9 liters of the filtrate obtained in Example 2 was adjusted to a pH of 5 with sodium hydroxide, and adsorbed to a 1 liter column of Amberlite XAD—4. The adsorbate was washed with water, and then eluted with a 50% aqueous solution of acetone. 2.3 liters of the active fraction was concentrated and dried under reduced pressure to obtain 19.6 g of a crude powder. The crude powder had an aminopeptidase B inhibitory activity ($IC_{50}$) of 0.2 μg/ml. Then, the crude powder was dissolved in a suitable amount of a 0.05 M aqueous solution of sodium chloride, and adsorbed to 500 ml of CM-Sephadex C-25. The adsorbate was washed with 1.5 liter of 0.05 mol aqueous solution of sodium chloride and 1 liter of 0.05 M citrate buffer solution (pH 4.5), and subjected to gradient elution with 2.5 liters of the same buffer solution along with 2.5 liters of the same buffer solution containing 0.55 M sodium chloride. According to this procedure, the eluate was collected as 18 ml fractions. Arphamenine A was obtained in Fraction Nos. 39 to 59, while Arphamenine B was obtained in Fraction Nos. 60 to 89. The respective fractions were desalted with Amberlite XAD—4, and then freeze-dried to obtain 188 mg of a crude Arphamenine A powder (aminopeptidase B inhibitory activity $IC_{50}$ = 0.0065 μg/ml) from the Arphamenine A fractions. From the Arphamenine B fractions was obtained a crude Arphamenine powder in a yield of 79 mg (aminopeptidase B inhibitory activity $IC_{50}$ = 0.0023 μg/ml).

Example 4

188 mg of the crude Arphamenine A powder obtained in Example 3 was dissolved in a suitable amount of a 0.05 M aqueous solution of sodium chloride, and adjusted to a pH of 2.3 with 1 N hydrochloric acid. The solution was adsorbed to an 80 ml column of CM-Sephadex C—25, and washed with 100 ml of a 0.07 M aqueous solution of sodium chloride. The adsorbate was then subjected to gradient elution using 300 ml of a 0.07 M aqueous solution of sodium chloride and 300 ml of a 0.5 M aqueous solution of sodium chloride. This purification step gave fractions (8 ml each), and Arphamenine was yielded in Fraction Nos. 11 to 34. The Fraction Nos. 11 to 30 were concentrated, and adjusted to a pH of 2.3 with 1 N hydrochloric acid. The concentrate was eluted with water on a 500 ml column of Sephadex LH—20 to desalt it. The desalted solution of Arphamenine was adjusted to a pH of 5 with Dowex WGR, and freeze-dried to obtain 94 mg of Arphamenine A as a white powder (aminopeptidase B inhibitory activity $IC_{50}$ = 0.0054 μg/ml).

Example 5

79 mg of the Arphamenine B fraction obtained in Example 3 was dissolved in a suitable amount of a 0.05 mol aqueous solution of sodium chloride. The solution was adjusted to a pH of 2.3 with 1 N hydrochloric acid, and adsorbed to a 100 ml column of CM-Sephadex C—25. The adsorbate was washed with 100 ml of a 0.15 M aqueous solution of sodium chloride, and then subjected to gradient elution using 350 ml of a 0.15 M aqueous solution of sodium chloride and 350 ml of a 0.6 M aqueous solution of sodium chloride. According to this purification step, the eluate was obtained as 11 ml fractions, and Arphamenine B was yielded in Fraction Nos. 17 to 30. These fractions were concentrated, and eluted with 0.01 N hydrochloric acid on a 500 ml column of Sephadex LH—20 to desalt the concentrate. The desalted fraction of Arphamenine B was adjusted to a pH of 5 with Dowes WGR, and freeze-dried to obtain 32 mg of Arphamenine B as a white powder. This product had an aminopeptidase B inhibitory activity, $IC_{50}$, of 0.0020 μg/ml.

Example 6

A 500 ml Sakaguchi flask was charged with 80 ml of a culture medium comprising 1.5% glycerin, 1.5% soluble starch, 0.5% Prorich, 1.5% fish meal, 0.2% calcium carbonate and 0.05% anti-foaming agent (KM—72). The culture medium was sterilized for 15 minutes at 120°C, cooled, and inoculated with a loopful of *Chromobacterium violaceum* BMG361—CF4 (Deposit No. 6521 at the Fermentation Research Institute, Agency of Industrial Science and Technology, Ministry of International Trade and Industry of Japan) cultivated by slant culture. The inoculum was shake-cultured at 28°C for 24 hours at 135 reciprocations per minute to make a primary inoculum.

A 100 liter tank was charged with 50 liters of a culture medium comprising 1.5% glycerin, 1.5% soluble starch, and 0.5% Prorich, 1.74% bonito extract, 0.2% calcium carbonate and 0.05% anti-foaming agent (KM—72). The culture medium was sterilized at 120°C for 30 minutes, cooled, and inoculated with 80 ml of the primary inoculum. The inoculated medium was cultivated for 24 hours at 28°C with stirring at 200 rpm while feeding sterilized air at a rate of 50 liters per minute, whereby a secondary inoculum was prepared.

300 liters of a culture medium comprising 3.0% soluble starch, 0.5% Prorich, 1.2% corn gluten meal, 0.2% calcium carbonate and 0.05% anti-foaming agent (KM—72) was put in a 570-liter tank, the culture medium was sterilized at 120°C for 30 minutes, cooled, and inoculated with 6 liters of the secondary inoculum. The inoculum was cultivated at 28°C for 24 hours with stirring at 190 rpm while feeding 300 liters of air per minute. After cultivation, the culture medium was adjusted to a pH of 2 with sulfuric acid, and suction-filtered using a filter aid (Hyflo Super Cell). The filtrate was adjusted to a pH of 6 with sodium hydroxide, and suction-filtered again to obtain 185 liters of a filtrate. This filtrate had an aminopeptidase B inhibitory activity, $IC_{50}$, of 0.17 µl/ml.

10 liters of carbon black for chromatography use was added to 185 liters of said filtrate, and the mixture was stirred for 2 hours. The carbon black was separated from the mixture through a 200-mesh sieve. The carbon black was then washed with 50 liters of water, and added to 80 liters of a 50% aqueous solution of acetone adjusted to a pH of 2 with hydrochloric acid. The mixture was stirred for 2 hours for extraction. The carbon black was removed by means of a basket centrifuge, and the extract was concentrated to obtain 3.54 liters of a concentrate. The aminopeptidase B inhibitory activity ($IC_{50}$) of the concentrate was 0.0063 µl/ml.

Example 7

3.54 liters of the concentrate obtained in Example 6 was neutralized with 2 N sodium hydroxide to make 5.35 liters of a liquid. The liquid was adsorbed to a 1.7 liter column of Amberlite XAD—4, and washed with water. The column was eluted with 12 liters of a 50% aqueous solution of acetone, and 7 liters of the active fraction was concentrated under reduced pressure to yield 280 ml of a concentrate. The aminopeptidase B inhibitory activity ($IC_{50}$) of the concentrate was 0.01 µl/ml. Then, the concentrate was adsorbed to a 1.7-liter column of CM-Sephadex, and washed with 6 liters of a 0.05 M aqueous solution of sodium chloride and 2 liters of 0.05 M citrate buffer solution (pH 4.5). The washed adsorbate was gradient-eluted with 6 liters of the same buffer solution and 6 liters of the same buffer solution containing 0.6 M sodium chloride. According to this procedure, the eluate was collected as fractions of 200 ml each. Arphamenine A appeared in Fraction Nos. 30 to 39, and Arphamenine B in Fraction Nos. 42 to 52. The respective fractions were concentrated and desalted with Amberlite XAD—4 to obtain 85 ml of a Arphamenine A fraction ($IC_{50}$ = 0.00058 µl/ml) and 43 ml of an Arphamenine B fraction ($IC_{50}$ = 0.00031 µl/ml).

Example 8

85 ml of the Arphamenine A fraction obtained in Example 7 was adsorbed to a 240 ml column of Biogel P—2 (a product of Bio-lad). The column was washed with 2.5 liters of water and 1 liter of a 20% aqueous solution of methanol, and eluted with a 0.001 M aqueous solution of sodium chloride. The eluate was collected as fractions of 18 ml each. Arphamenine A was seen in Fraction Nos. 7 to 81. These fractions were concentrated, desalted with Amberlite XAD—4, and freeze-dried to obtain 1.48 g of Arphamenine A as a light yellow powder. This powder had an aminopeptidase B inhibitory activity ($IC_{50}$) of 0.030 µg/ml.

Example 9

43 ml of the Arphamenine B fraction obtained in Example 7 was adsorbed to a 240 ml column of Biogel P—2 (Bio-lad's product). The column was washed with 2 liters of water and 1 liter of a 20% aqueous solution of methanol, followed by eluting it with a 0.005 M aqueous solution of sodium chloride. The eluate was collected as fractions of each 18 ml, and Arphamenine B was seen in Fraction Nos. 6 to 25. These fractions were concentrated, desalted with Amberlite XAD—4, and freeze-dried. There was obtained 1.89 g of Arphamenine B as a light yellow powder. The aminopeptidase B inhibitory activity ($IC_{50}$) of the powder was 0.019 µg/ml.

Brief Description of the Drawings:

Fig. 1 shows the ultraviolet absorption spectrum of Arphamenine A according to this invention.

Fig. 2 shows the infrared absorption spectrum of Arphamenine A.

Fig. 3 shows the nuclear magnetic resonance absorption spectrum of Arphamenine A.

Fig. 4 shows the ultraviolet absorption spectrum of Arphamenine B claimed in this invention.

Fig. 5 illustrates the infrared absorption spectrum of Arphamenine B.
Fig. 6 shows the nuclear magnetic resonance absorption spectrum of Arphamenine B.

**Claims for the Contracting States: BE CH DE FR GB IT LI NL SE**

1. The novel microorganism strain Chromobacterium violaceum FERM—P—6521.
2. A compound of the formula I

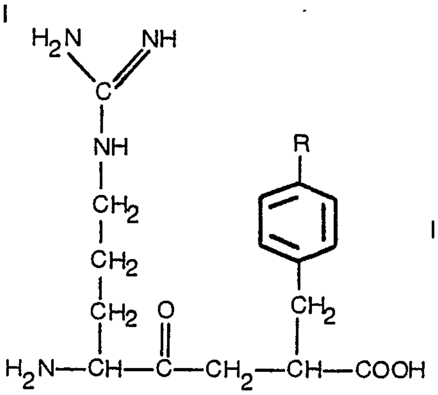

I

wherein R represents hydrogen or the hydroxyl group, and physiologically acceptable salts thereof.
3. A process for producing a compound of the formula I as claimed in claim 2 which comprises cultivating microorganisms of the genus Chromobacterium capable of producing the compound of formula I in the presence of known nutrients and recovering the compound of the formula I from the culture medium.
4. The process as claimed in claim 3 wherein the strain Chromobacterium violaceum FERM—P—6521 as claimed in claim 1 is used.
5. The process as claimed in claims 3 and 4 wherein a liquid culture is used and the compounds of the formula I are recovered from the culture by adsorbing a filtrate of the culture medium to an usual adsorbent and isolating the desired compounds from the adsorbate.
6. A pharmaceutical preparation comprising as active ingredient a compound of the formula I or a physiologically acceptable salt thereof as claimed in claim 2 in association with a pharmaceutically acceptable carrier and/or stabilizer.
7. A process for the preparation of a pharmaceutical preparation characterized in that a compound of the formula I and/or one of its physiologically acceptable salts is brought together with at least one solid, liquid or semi-liquid carrier and/or stabilizer into a suitable dosage form.
8. A compound of the formula I and/or one of its physiologically acceptable salts for use as medicament.

**Claims for the Contracting State: AT**

1. A process for producing a compound of the formula I

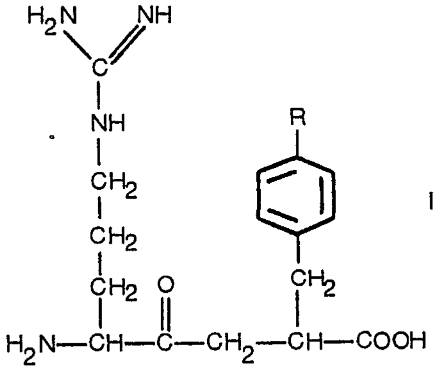

I

wherein R represents hydrogen or the hydroxyl group, which comprises cultivating microorganisms of the genus Chromobacterium capable of producing the compound of formula I in the presence of known nutrients and recovering the compound of the formula I from the culture medium as such or as a physiologically acceptable salt thereof.
2. The process as claimed in claim 1 wherein the strain Chromobacterium violaceum FERM—P—6521 is used.
3. The process as claimed in claims 1 and 2 wherein a liquid culture is used and the compounds of the formula I are recovered from the culture by adsorbing a filtrate of the culture medium to an usual adsorbent and isolating the desired compounds from the adsorbate.

4. A process for the preparation of a pharmaceutical preparation characterized in that a compound of the formula I and/or one of its physiologically acceptable salts is brought together with at least one solid, liquid or semi-liquid carrier and/or stabilizer into a suitable dosage form.

**Patentansprüche für die Vertragsstaaten: BE CH DE FR GB IT LI NL SE**

1. Neuer Mikroorganismusstamm Chromobacterium violaceum FERM—P—6521.

2. Verbindung der Formel I

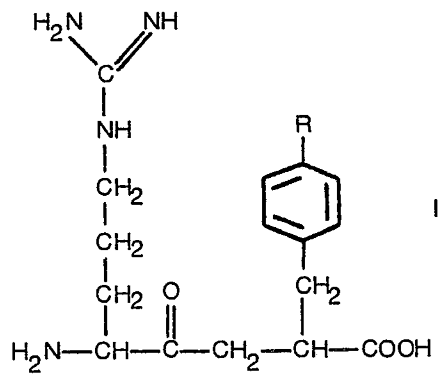

I

worin R Wasserstoff oder die Hydroxygruppe bedeutet, und deren physiologisch unbedenkliche Salze.

3. Verfahren zur Herstellung einer Verbindung der Formel I nach Anspruch 2, dadurch gekennzeichnet, dass Mikroorganismen der Gattung Chromobacterium, die die Verbindung der Formel I in Gegenwart bekannter Nährstoffe bilden können, gezüchtet werden und dass die Verbindung der Formel I aus dem Nährmedium gewonnen wird.

4. Verfahren nach Anspruch 3, wobei der Stamm Chromobacterium violaceum FERM—P—6521 nach Anspruch 1 verwendet wird.

5. Verfahren nach Anspruch 3 und 4, wobei ein flüssiges Nährmedium verwendet wird und die Verbindungen der Formel I durch Adsorption eines Filtrates des Nährmediums an einem üblicherweise verwendeten Adsorptionsmittel und durch Isolierung der gewünschten Verbindungen aus dem Adsorbat gewonnen werden.

6. Pharmazeutisches Präparat mit einer Verbindung der Formel I als Wirkstoff oder einem ihrer physiologisch unbedenklichen Salz nach Anspruch 2 in Verbindung mit einem pharmazeutisch unbedenklichen Trägerstoff und/oder Stabilisator.

7. Verfahren zur Herstellung eines pharmazeutischen Präparates, dadurch gekennzeichnet, dass eine Verbindung der Formel I und/oder eines ihrer physiologisch unbedenklichen Salze zusammen mit mindestens einem festen, flüssigen oder halbflüssigen Trägerstoff und/oder Stabilisator in eine geeignete Dosierungsform gebracht wird.

8. Verbindung der Formel 1 und/oder eines ihrer physiologisch unbedenklichen Salze zur Verwendung als Medikament.

**Patentansprüche für den Vertragstaat: AT**

1. Verfahren zur Herstellung einer Verbindung der Formel I

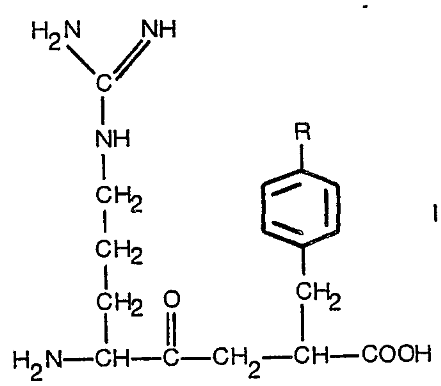

I

worin R Wasserstoff oder die Hydroxygruppe bedeutet, dadurch gekennzeichnet, dass Mikroorganismen der Gattung Chromobacterium, die die Verbindung der Formel I in Gegenwart bekannter Nährstoffe bilden können, gezüchtet werden und dass die Verbindung der Formel I als solche oder in Form eines ihrer physiologisch unbedenklichen Salze aus dem Nährmedium gewonnen wird.

2. Verfahren nach Anspruch 1, wobei der Stamm Chromobacterium violaceum FERM-P-6521 verwendet wird.

3. Verfahren nach Anspruch 1 und 2, wobei ein flüssiges Nährmedium verwendet wird und die Verbindungen der Formel I durch Adsorption eines Filtrates des Nährmediums an einem üblicherweise verwendeten Adsorptionsmittel und durch Isolierung der gewünschten Verbindungen aus dem Adsorbat gewonnen werden.

4. Verfahren zur Herstellung eines pharmazeutischen Präparates, dadurch gekennzeichnet, dass eine Verbindung der Formel I und/oder eines ihrer physiologisch unbedenklichen Salze zusammen mit mindestens einem festen, flüssigen oder halbflüssigen Trägerstoff und/oder Stabilisator in eine geeignete Dosierungsform gebracht wird.

**Revendications pour les Etats contractants: BE CH DE FR GB IT LI NL SE**

1. Nouvelle souche de microorganisme Chromobacterium violaceum FERM—P—6521.

2. Composé de formule I

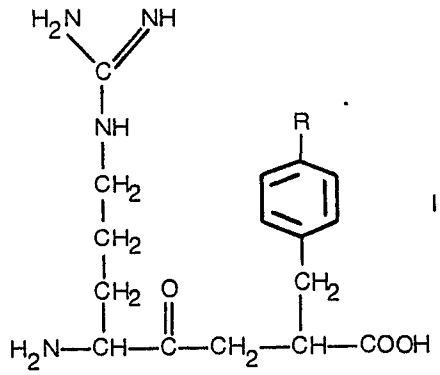

I

dans laquelle R représente un atome d'hydrogène ou le groupe hydroxyle, et les sels physiologiquement acceptables de celui-ci.

3. Procédé de production d'un composé de formule I suivant la revendication 2, dans lequel on cultive des microorganismes du genre Chromobacterium susceptibles de produire le composé de formule I en présence de produits nutritifs connus, et on récupère le composé de formule I à partir du milieu de culture.

4. Procédé suivant la revendication 3, dans lequel on utilise la souche Chromobacterium violaceum FERM—P—6521 revendiquée dans la revendication 1.

5. Procédé suivant les revendications 3 et 4, dans lequel on utilise une culture liquide et les composés de formule I sont récupérés à partir de la culture par adsorption d'un filtrat du milieu de culture sur un adsorbant habituel, et on isole les composés désirés à partir de l'adsorbat.

6. Préparation pharmaceutique comprenant comme ingrédient actif, un composé de formule I ou un sel physiologiquement acceptable de celui-ci tel que revendiqué dans la revendication 2 en association avec un véhicule pharmaceutiquement acceptable et/ou un agent stabilisant.

7. Procédé de préparation d'un composition pharmaceutique, caractérisé en ce que l'on réunit un composé de formule I et/ou un de ses sels physiologiquement acceptables avec au moins un véhicule solide, liquide ou semi-liquide, et/ou un agent stabilisant pour obtenir une forme de base appropriée.

8. Composé de formule I et/ou un de ses sels physiologiquement acceptables pour utilisation comme médicament.

**Revendications pour l'Etat contractant: AT**

1. Procédé de production d'un composé de formule

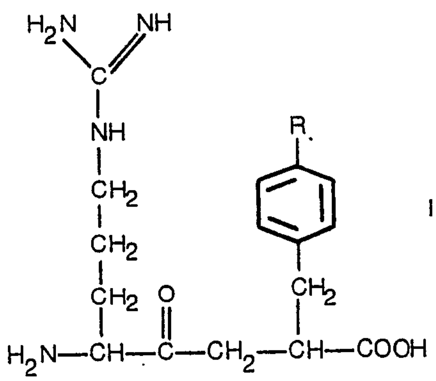

I

dans laquelle R représente un atome d'hydrogène ou le groupe hydroxyle, dans lequel on cultive des microorganismes du genre Chromobacterium susceptibles de produire le composé de formule I en présence de produits nutritifs connus, et on récupère le composé de formule I à partir du milieu de culture tel quel ou sous la forme d'un de ses sels physiologiquement acceptables.

2. Procédé suivant la revendication 1, dans lequel on utilise la souche Chromobacterium violaceum FERM—P—6521.

3. Procédé suivant les revendications 1 et 2, dans lequel on utilise une culture liquide et on récupère les composés de formule I à partir de la culture par adsorption d'un filtrat du milieu de culture sur un adsorbant classique, et on isole les composés requis à partir de l'adsorbat.

4. Procédé de préparation d'une composition pharmaceutique, caractérisé en ce qu'on réunit un composé de formule I et/ou un de ses sels physiologiquement acceptables avec au moins un véhicule solide, liquide ou semi-liquide, et/ou un agent stabilisant pour obtenir une forme de dose appropriée.

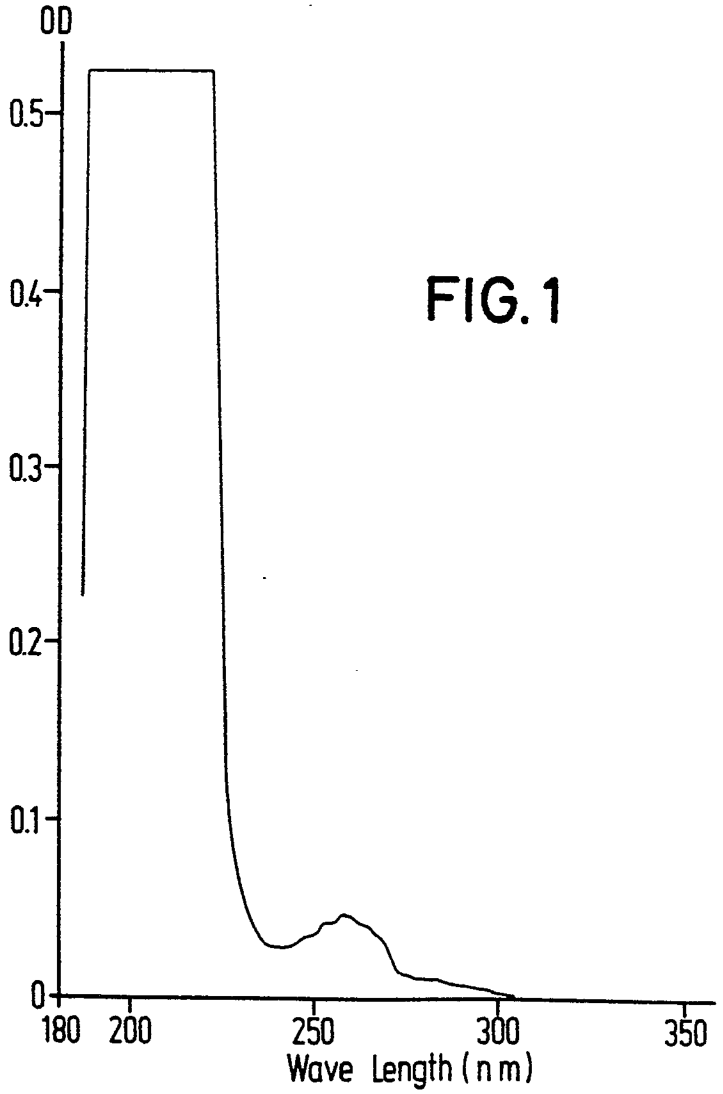


FIG. 1

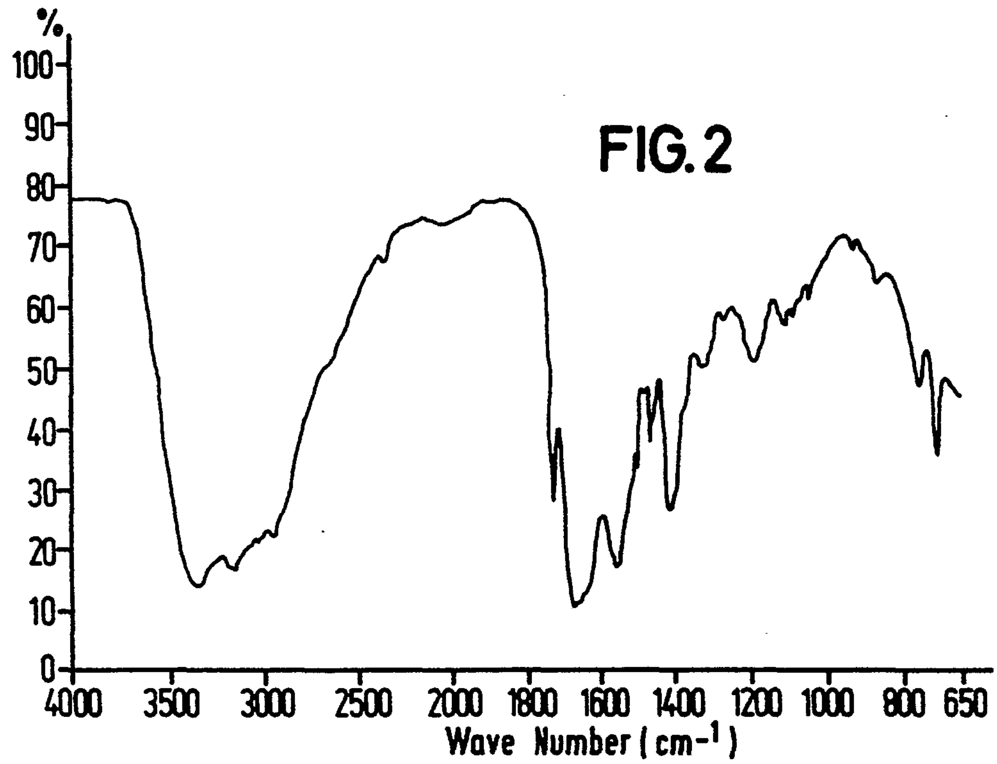
%
100
90
80
70
60
50
40
30
20
10
0
4000
3500
3000
2500
2000
1800
1600
1400
1200
1000
800
650
Wave Number ( cm-1 )

FIG. 2

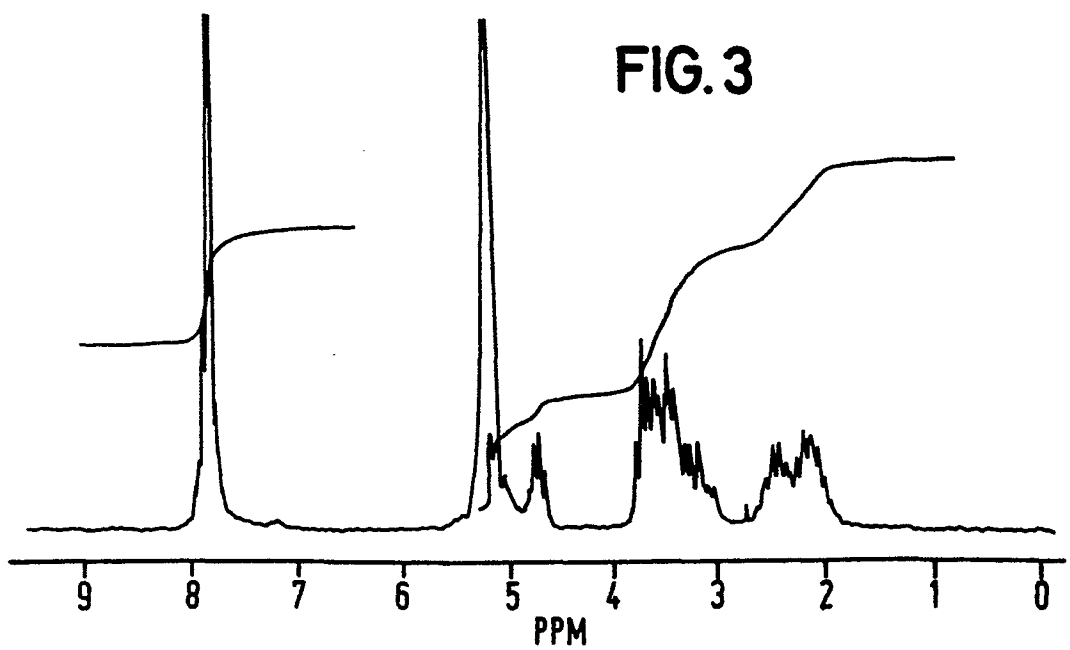
9
8
7
6
5
4
3
2
1
0
PPM

FIG. 3

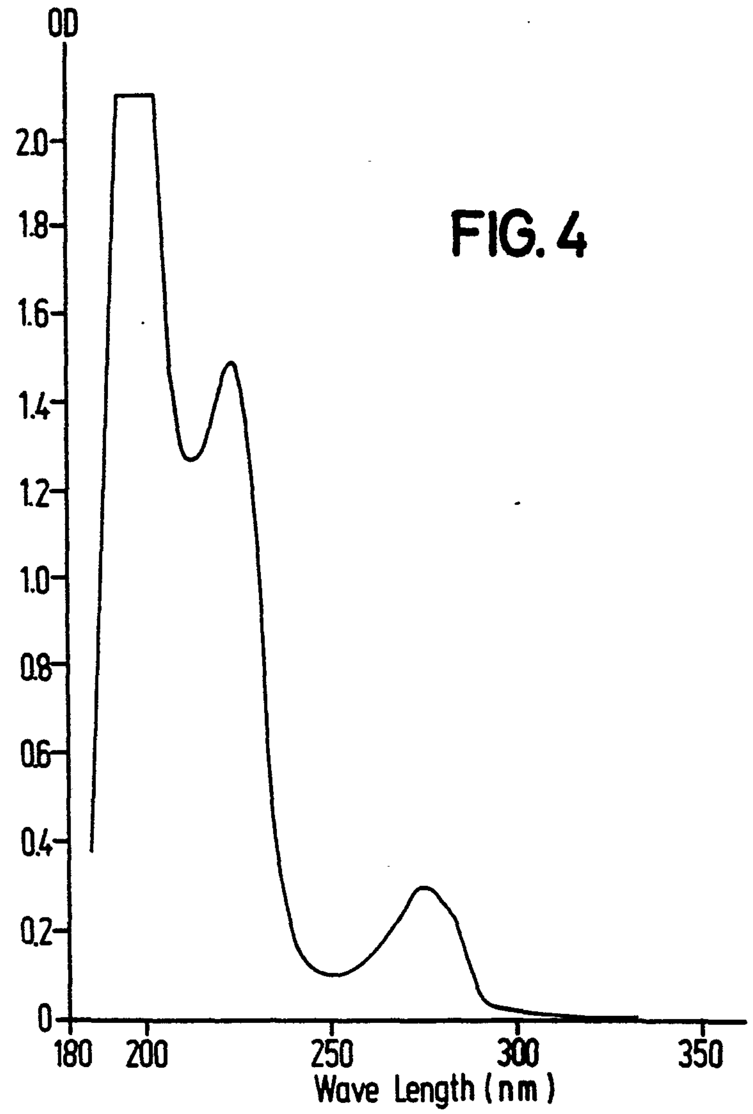
OD
2.0
1.8
1.6
1.4
1.2
1.0
0.8
0.6
0.4
0.2
0
180
200
250
300
350
Wave Length (nm)

FIG. 4

FIG. 5

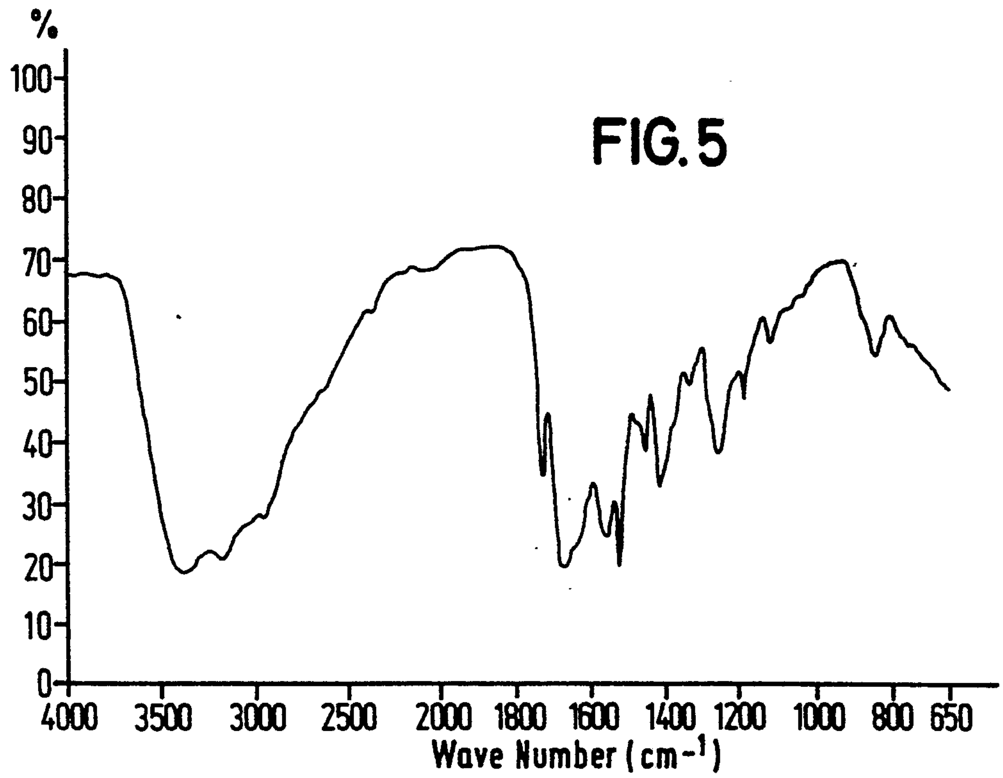
%
100
90
80
70
60
50
40
30
20
10
0
4000
3500
3000
2500
2000
1800
1600
1400
1200
1000
800
650
Wave Number (cm-1)

FIG. 6

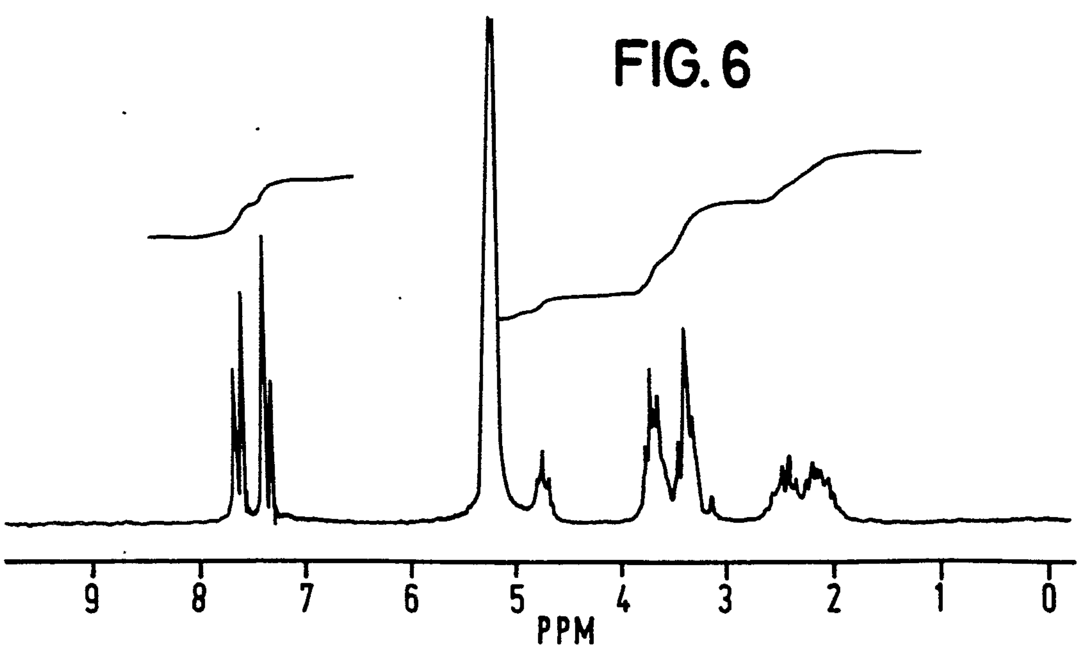
9
8
7
6
5
4
3
2
1
0
PPM